(19) Europäisches Patentamt / European Patent Office / Office européen des brevets

(11) **EP 2 833 938 B1**

(12) **EUROPÄISCHE PATENTSCHRIFT**

(45) Veröffentlichungstag und Bekanntmachung des Hinweises auf die Patenterteilung:
**27.06.2018 Patentblatt 2018/26**

(21) Anmeldenummer: **13715660.0**

(22) Anmeldetag: **04.04.2013**

(51) Int Cl.:
*A61M 1/14* *(2006.01)*       *G01N 27/06* *(2006.01)*
*G01N 21/41* *(2006.01)*      *A61M 1/16* *(2006.01)*
*A61M 1/28* *(2006.01)*       *G01N 27/08* *(2006.01)*

(86) Internationale Anmeldenummer:
**PCT/EP2013/057099**

(87) Internationale Veröffentlichungsnummer:
**WO 2013/150096 (10.10.2013 Gazette 2013/41)**

(54) **VERFAHREN UND VORRICHTUNG ZUR BESTIMMUNG DER ZUSAMMENSETZUNG MEDIZINISCHER FLÜSSIGKEITEN BEZÜGLICH IHRES ANTEILS AN ELEKTROLYTEN UND NICHTELEKTROLYTEN**

METHOD AND APPARATUS FOR DETERMINING THE COMPOSITION OF MEDICAL FLUIDS WITH RESPECT TO THEIR CONTENT OF ELECTROLYTES AND NONELECTROLYTES

MÉTHODE ET APPAREIL POUR DÉTERMINER LA COMPOSITION DE FLUIDES MÉDICAUX EN FONCTION DE LEUR TENEUR EN ÉLECTROLYTES ET NON ÉLECTROLYTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.04.2012 DE 102012103010**
**05.04.2012 US 201261620493 P**

(43) Veröffentlichungstag der Anmeldung:
**11.02.2015 Patentblatt 2015/07**

(73) Patentinhaber: **Fresenius Medical Care Deutschland GmbH**
**61352 Bad Homburg (DE)**

(72) Erfinder:
• **NIER, Volker**
**61203 Reichelsheim (DE)**
• **WÜPPER, Andreas**
**64572 Büttelborn (DE)**

(74) Vertreter: **Nordmeyer, Philipp Werner**
**df-mp Dörries Frank-Molnia & Pohlman**
**Patentanwälte Rechtsanwälte PartG mbB**
**Theatinerstraße 16**
**80333 München (DE)**

(56) Entgegenhaltungen:
**EP-A1- 1 547 629      WO-A1-96/25214**
**JP-A- H1 085 573      JP-A- 2012 026 912**
**US-A- 5 762 769**

• **"Refraktometrie" In: Helm, Laszlo: "Fachlexikon ABC Messtechnik", 1985, Verlag Harri Deutsch, Thun und Frankfurt/Main, XP002698205, ISBN: 3 87144 846 X Seiten 387-388, Seite 387, rechte Spalte, Zeile 7 - Seite 388, linke Spalte, Zeile 7; Tabelle 1**

## Beschreibung

Technisches Gebiet

Die vorliegende Erfindung betrifft ein Verfahren sowie eine Vorrichtung zur Bestimmung der Zusammensetzung medizinischer Flüssigkeiten bezüglich ihres Anteils an Elektrolyten und Nichtelektrolyten. Bevorzugt werden dabei Dialysate zur Verwendung bei der Hämodialyse und bei der Peritonealdialyse analysiert.

Stand der Technik

Bei der Herstellung medizinischer Flüssigkeiten ist es häufig notwendig, bestimmte Verhältnisse von Elektrolyten und Nichtelektrolyten in der jeweiligen Flüssigkeit einzustellen und die konkrete Einstellung der jeweiligen Verhältnisse zu überwachen.

Beispielsweise ist es im Bereich der Dialyse bekannt, beispielsweise bei der Hämodialyse, Hämofiltration, Hämodiafiltration und Peritonealdialyse, medizinische Flüssigkeiten als Dialysat oder als Substitutionsflüssigkeit zu verwenden, welche sowohl Elektrolyte als auch Nichtelektrolyte umfassen.

Häufig werden die entsprechenden medizinischen Flüssigkeiten vor der Anwendung in den jeweiligen Dialyseverfahren online oder in Tanks gemischt, beispielsweise durch Hinzufügen von entsprechenden Konzentraten von Elektrolyten und Nichtelektrolyten zu Wasser.

Bei der Mischung der entsprechenden Konzentrate mit Wasser ist darauf zu achten, dass die vorgesehenen Endkonzentrationen in der jeweiligen medizinischen Flüssigkeit erreicht werden, um eine hohe Patientensicherheit zu garantieren. Hierzu ist es bekannt, das Dialysat in den Dialysemaschinen durch kontinuierliche Messung der Leitfähigkeit zu überwachen.

Gebrauchsfertige Lösungen der medizinischen Flüssigkeit müssen beide Komponenten, nämlich Elektrolyte sowie Nichtelektrolyte, in vorgeschriebenen Konzentrationen enthalten. Eine automatische Überwachung der vorgesehenen Endkonzentrationen in der medizinischen Flüssigkeit wird durchgeführt, bevor das Dialysat zur Behandlung des Patienten verwendet wird.

Da die Konzentrationen in dem herzustellenden Dialysat festgelegt sind, ist auch dessen elektrische Leitfähigkeit definiert. Wenn die elektrische Leitfähigkeit während des Betriebs von dem vorgegebenen Zielwert abweicht, dann liegt eine Störung der Herstellung des Dialysats vor und die entsprechende Dialysesitzung muss gestoppt oder unterbrochen werden.

Weiterhin kann es trotz des Zuführens der korrekten Mengen an Elektrolytkonzentrat sowie Nichtelektrolytkonzentrat vorkommen, dass die entsprechenden Konzentrate, beispielsweise wenn sie in Trockenform vorliegen, nicht vollständig mit der Flüssigkeit gemischt werden beziehungsweise in Lösung treten. Der entsprechende Effekt ist der gleiche, nämlich, dass das zu verwendende Dialysat nicht die vorgegebenen Konzentrationsverhältnisse aufweist.

Aus der US 5,762,769 ist ein Verfahren zum Messen der Konzentration von Nichtelektrolyten in einer Elektrolytlösung bekannt, wobei die gemischte Elektrolytlösung, welche zumindest einen Elektrolyten und einen Nichtelektrolyten umfasst, über eine elektrische Leitfähigkeitsmessung gemessen wird.

Aus der europäischen Anmeldung EP 1 547 629 A1 ist ein Verfahren bekannt, welches ein Überwachen eines Peritonealdialyseverfahren ermöglicht und bei dem die Konzentration eines osmotischen Agens gemessen wird. Die Wirkung und Effektivität des Agens können entsprechend der Messung der Konzentration des Agens durch Ultrafiltration kontrolliert und erhöht werden.

Die japanische Anmeldung JP 2012 026912 A offenbart ein Verfahren, bei dem nach einer Verdünnungsreihe eine Salzkonzentration gemessen wird, wobei die unverdünnte Salzkonzentration anhand einer gemessenen, verdünnten, Salzkonzentration und einem Verdünnungsfaktor hochgerechnet werden kann.

Die japanische Anmeldung JP H10 85573 A offenbart eine kontrollierte, gestufte Herstellung einer Flüssigkeit mittels einer Messung der elektrischen Leitfähigkeit und einer glukosebedingter Korrelation.

Darstellung der Erfindung

Entsprechend ist es eine Aufgabe der vorliegenden Erfindung, die Bestimmung des Anteils von Elektrolyt und Nichtelektrolyt in einer medizinischen Flüssigkeit weiter zu verbessern.

Diese Aufgabe wird mit einem Verfahren mit den Merkmalen des Anspruchs 1 gelöst. Vorteilhafte Weiterbildungen ergeben sich aus den abhängigen Unteransprüchen.

Entsprechend umfasst das Verfahren zur Bestimmung der Zusammensetzung medizinischer Flüssigkeiten bezüglich ihres Anteils an Elektrolyten und Nichtelektrolyten die Schritte des Bestimmens mindestens einer ersten physikalischen Größe der medizinischen Flüssigkeit zur eindeutigen Bestimmung des Anteils an Nichtelektrolyten in der medizinischen Flüssigkeit, das simultane Bestimmen mindestens einer zweiten physikalischen Größe der medizinischen Flüssigkeit zur Bestimmung des Anteils an Elektrolyten in der medizinischen Flüssigkeit, und das Bestimmen des Anteils an Elektrolyten und Nichtelektrolyten in der medizinischen Flüssigkeit auf Grundlage der ersten und der zweiten physikalischen Größe.

Dadurch, dass mindestens eine erste physikalische Größe der medizinischen Flüssigkeit bestimmt wird, welche zur eindeutigen Bestimmung des Anteils an Nichtelektrolyten dient, und simultan dazu eine zweite physikalische Größe der medizinischen Flüssigkeit bestimmt wird, welche den Anteil an Elektrolyten bestimmt, wird es möglich, den Einfluss des Nichtelektrolyten aus der physikalischen Größe für den Elektrolyten herauszurechnen beziehungsweise diese Größe bei der Bestimmung der Elektrolytkonzentration zu berücksichtigen. Als erste physikalische Größe zur eindeutigen Bestimmung des Anteils an Nichtelektrolyten in der medizinischen Flüssigkeit wird der Brechungsindex der medizinischen Flüssigkeit bestimmt. Der Brechungsindex einer medizinischen Flüssigkeit und insbesondere eines Dialysats ist im Wesentlichen unabhängig von der Konzentration der Elektrolyte. Dies ist besonders der Fall für den Konzentrationsbereich der Elektrolyte, welcher für Dialysate relevant ist. Als zweite physikalische Größe der medizinischen Flüssigkeit, welche zur Bestimmung des Anteils an Elektrolyten dient, wird bevorzugt die Leitfähigkeit der medizinischen Flüssigkeit verwendet. Leitfähigkeitsmessungen an Dialysaten sind zur Bestimmung des Elektrolytgehaltes innerhalb des Dialysats wohl bekannt. Durch das angegebene Verfahren kann allerdings der Effekt des Nichtelektrolyten auf die Leitfähigkeit berücksichtigt werden. Aus der ersten physikalischen Größe wird der Anteil des Nichtelektrolyten eindeutig bestimmt und diese Bestimmung des Anteils des Nichtelektrolyten geht in die Bestimmung des Anteils des Elektrolyten auf Grundlage der zweiten physikalischen Größe mit ein, wobei bevorzugt der Effekt des Nichtelektrolyten auf die Messung der zweiten physikalischen Größe bei der Bestimmung des Anteils an Elektrolyten berücksichtigt wird.

Um ein Unterscheidungskriterium bereit zu stellen kann ein Zielwert oder ein Zielwertbereich für die erste physikalische Größe und/oder ein Zielwert oder ein Zielwertbereich für die zweite physikalische Größe vorbestimmt werden, und bei einer Abweichung der gemessenen ersten physikalischen Größe und/oder der zweiten physikalischen Größe von dem jeweiligen vorgegebenen Zielwert beziehungsweise Zielwertbereich kann ein Alarm ausgelöst werden und/oder eine Behandlung abgebrochen werden. Eine Vereinfachung des Verfahrens kann entsprechend dadurch erreicht werden, dass eine medizinische Flüssigkeit als für den jeweiligen Anwendungsfall geeignet bewertet wird, wenn sowohl für die erste physikalische Größe, bevorzugt den Brechungsindex, sowie die zweite physikalische Größe, bevorzugt die Leitfähigkeit, jeweils einen Zielwert oder Zielwertbereich einhalten. Wenn die jeweiligen Zielwerte Zielwertbereiche von der medizinischen Flüssigkeit eingehalten werden, so kann davon ausgegangen werden, dass sowohl der Anteil für die Elektrolyte als auch der Anteil für die Nichtelektrolyte in der jeweiligen medizinischen Flüssigkeit den Vorgaben entspricht. Eine besondere Bewertung beziehungsweise ein Herausrechnen des Effekts der Nichtelektrolyte auf die gemessene physikalische Größe der Elektrolyte kann auf diese Weise verzichtet werden. Die entsprechend hinterlegten Zielwerte für die erste und die zweite physikalische Größe entsprechen den Werten, welche in einer geeigneten medizinischen Flüssigkeit gemessen wurden.

Weiter bevorzugt ist es, die Temperatur der medizinischen Flüssigkeit zu messen und die Temperatur bei der Bestimmung des Anteils an Elektrolyten und Nichtelektrolyten auf Grundlage der ersten und zweiten physikalischen Größe zu berücksichtigen. Dies kann die Genauigkeit der Bestimmungen der Konzentrationsverhältnisse noch einmal weiter verbessern. Die oben gestellte Aufgabe wird weiterhin durch eine Vorrichtung mit den Merkmalen des Anspruchs 6 gelöst. Vorteilhafte Weiterbildungen ergeben sich aus den Unteransprüchen. Entsprechend ist eine Vorrichtung zur Bestimmung der Zusammensetzung medizinischer Flüssigkeiten bezüglich ihres Anteils an Elektrolyten und Nichtelektrolyten angegeben, welche eine erste Messvorrichtung zum Messen einer ersten physikalischen, dem Brechungsindex, der medizinischen Flüssigkeit, welche in einem eindeutigen Verhältnis zu dem Nichtelektrolyten in der medizinischen Flüssigkeit steht, umfasst. Eine zweite Messvorrichtung zur Bestimmung mindestens einer zweiten physikalischen Größe der medizinischen Flüssigkeit, welche in einem Verhältnis zu dem Anteil an Elektrolyten in der medizinischen Flüssigkeit steht, ist vorgesehen. Weiterhin ist eine Auswertungsvorrichtung zum Bestimmen des Anteils an Elektrolyten und Nichtelektrolyten in der medizinischen Flüssigkeit auf Grundlage der Messwerte der ersten Messvorrichtung und der zweiten Messvorrichtung vorgesehen.

Es ist von Vorteil, in einer Dialysevorrichtung das verwendete Dialysat beziehungsweise die verwendete Ersatzflüssigkeit beziehungsweise Substitutionsflüssigkeit mittels dem vorbeschriebenen Verfahren beziehungsweise mittels der vorbeschriebenen Vorrichtung bezüglich der Anteile an Elektrolyten und an Nichtelektrolyten zu überwachen. Hierzu ist es von Vorteil, die Überwachung derart zu vereinfachen, dass Zielwerte sowohl für die erste physikalische Größe, über welche eindeutig auf die Konzentration der Nichtelektrolyte geschlossen werden kann, als auch für die zweite physikalische Größe, über welche auf die Konzentration der Elektrolyte geschlossen werden kann, anzugeben. Solange sich die an das zu verwendenden Dialysat geforderten Werte im Bereich der jeweiligen Zielwerte bewegen, ist das Dialysat zur Verwendung bei der Behandlung des Patienten geeignet. Auf diese Weise kann die Patientensicherheit sichergestellt werden. Sobald einer der Werte den Zielwert beziehungsweise einen Zielwertbereich verlässt, wird die weitere Verwendung des Dialysats gestoppt und die Dialyse angehalten.

Dieses Verfahren ist sowohl bei Hämodialyse, Hämofiltrations- als auch Hämodiafiltrationsverfahren geeignet, und kann besonders bevorzugt im Bereich der Peritonealdialyse verwendet werden. Hier wird das Einströmen beziehungsweise Austauschen des Dialysats gestoppt, sobald eine der gemessenen physikalischen Größen den Zielwert beziehungsweise den Zielwertbereich verlässt. Gerade bei der Peritonealdialyse werden auch hohe Konzentrationswerte für die Nichtelektrolyte erreicht, so dass hier eine besonders genaue Überwachung mit dem vorgeschlagenen Verfahren und der vorgeschlagenen Vorrichtung erreicht werden kann.

## Kurze Beschreibung der Figuren

Bevorzugte weitere Ausführungsformen und Aspekte der vorliegenden Erfindung werden durch die nachfolgende Beschreibung der Figuren näher erläutert. Dabei zeigen:

Figur 1      Messungen der Leitfähigkeit einer medizinischen Flüssigkeit in Abhängigkeit vom prozentualen Anteil gelöster Elektrolyte bei verschiedenen prozentualen Anteilen eines gelösten Nichtelektrolyten;

Figur 2      Messungen der Leitfähigkeit einer medizinischen Flüssigkeit in Abhängigkeit vom prozentualen Anteil der gelösten Elektrolyten bei verschiedenen prozentualen Anteilen eines anderen gelösten Nichtelektrolyten;

Figur 3      Messungen des Brechungsindex einer medizinischen Flüssigkeit in Abhängigkeit vom prozentuellen Anteil eines gelösten Elektrolyten bei verschiedenen prozentualen Anteilen eines gelösten Nichtelektrolyten;

Figur 4      Messungen des Brechungsindex einer medizinischen Flüssigkeit in Abhängigkeit vom prozentualen Anteil eines gelösten Elektrolyten bei verschiedenen prozentualen Anteilen eines anderen gelösten Nichtelektrolyten;

Figur 5      Messungen des Brechungsindex einer medizinischen Flüssigkeit in Abhängigkeit vom prozentualen Anteil eines gelösten Nichtelektrolyten bei verschiedenen prozentualen Anteilens eines gelösten Elektrolyten;

Figur 6      Messungen des Brechungsindex einer medizinischen Flüssigkeit in Abhängigkeit vom prozentualen Anteil eines anderen gelösten Nichtelektrolyten bei verschiedenen prozentualen Anteilen eines gelösten Elektrolyten; und

Figur 7      den schematischen Aufbau einer Hämodialysevorrichtung.

## Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente mit identischen Bezugszeichen bezeichnet und auf eine wiederholte Beschreibung dieser Elemente wird teilweise verzichtet, um Redundanzen in der Beschreibung zu vermeiden.

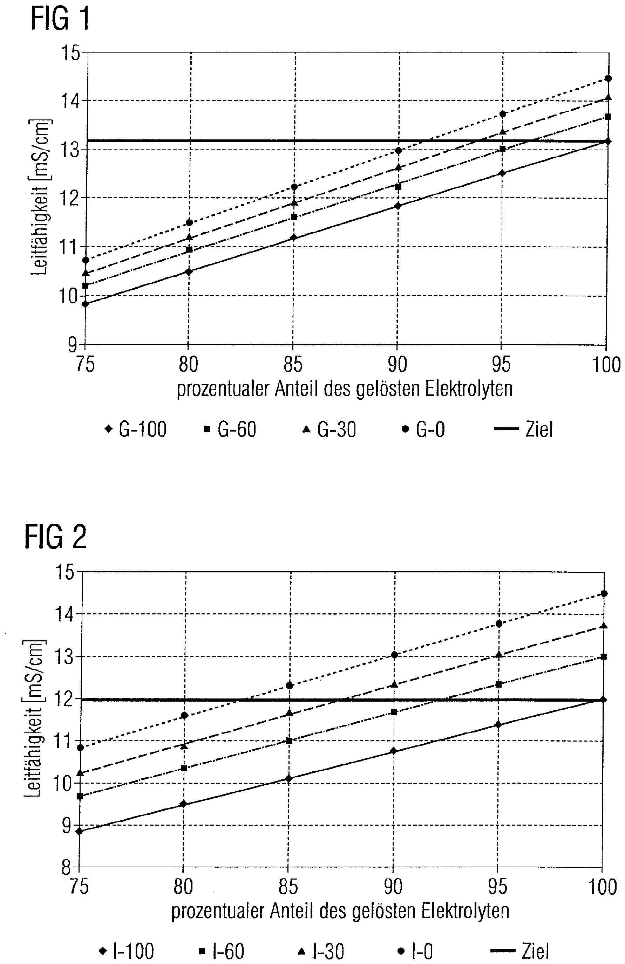

In den Figuren 1 und 2 sind die Ergebnisse von Leitfähigkeitsmessungen an einer medizinischen Flüssigkeit gezeigt, wobei die Leitfähigkeit in mS/cm gegenüber dem prozentualen Anteil des gelösten Elektrolyten angegeben wird. Die in den Diagrammen angegebenen Konzentrationen sind in Prozentwerten der jeweils höchsten Konzentrationen angegeben. Die maximalen Konzentrationen stellen dabei die Werte einer gebrauchsfähigen Lösung dar, welche den üblichen Konzentrationen von Peritonealdialyselösungen entspricht. Die maximalen Konzentrationen sind hier beispielsweise 140 mmol/l NaCl sowie 42,5 g/l Glukose beziehungsweise 75 g/l Icodextrin. Die Verwendung unterschiedlicher Konzentrationen spiegelt den möglichen Fall wider, dass eine vollständige Auflösung eines Trockenkonzentrats nicht erreicht werden kann, oder dass die jeweiligen Dosiervorrichtungen, welche die Elektrolyte und die Nichtelektrolyte in die medizinische Flüssigkeit dosieren, nicht korrekt arbeiten.

In den vorliegenden Messungen war der Elektrolyt durch NaCl gegeben, wobei die angegebenen 100 % einer Konzentration von 140 mmol/l entsprechen, also dem physiologisch richtigen Wert für NaCl im Blut beziehungsweise im Blutplasma. Die übrigen Prozentwerte des Elektrolyten in den Messungen entsprechen Konzentrationen von 105 mmol/l (75 %), 112 mmol/l (80 %), 119 mmol/l (85 %), 126 mmol/l (90 %) und 133 mmol/l (95 %).

Zusätzlich zu dem Elektrolyten NaCl wurde der jeweiligen zu messenden medizinischen Flüssigkeit auch ein Nichtelektrolyt hinzugefügt. In der in Figur 1 gezeigten Messung wurde Glukose als Nichtelektrolyt verwendet. 100 % Glukose entsprechen dabei einer Konzentration von 42,5 g/l, entsprechend der im Bereich der Peritonealdialyse (PD) üblicher Weise verwendeten Glucosekonzentration. Es wurden Messungen für 100 %, 60 %, 30 % und 0 % Glukoseanteil aufgenommen, wobei diese Anteile Konzentrationen von 42,5 g/l, 25,5 g/l, 12,75 g/l und 0 g/l Glukose entsprechen. Die entsprechenden Graphen sind in Figur 1 mit G-100, G-60, G-30 und G-0 gekennzeichnet.

In Figur 2 wurde als Nichtelektrolyt Icodextrin verwendet, wobei hier als 100% eine Konzentration von 75 g/l verwendet wurde - ebenfalls entsprechend dem in der Peritonealdialyse üblicher Weise verwendeten Wert. Die übrigen Konzentrationen liegen entsprechend bei 45 g/l, 22,5 g/l und 0 g/l und sind analog wie in Figur 1 gekennzeichnet durch I-100, I-60, I-30 und I-0.

Die Leitfähigkeit in den jeweiligen Messungen wurde mittels eines handelsüblichen Leitfähigkeitsmessers, nämlich des "Ionometer 3" von Fresenius Medical Care, gemessen.

Aus den Figuren 1 und 2 lässt sich sofort erkennen, dass die Leitfähigkeit der jeweiligen medizinischen Flüssigkeiten proportional zur Elektrolytkonzentration ist. Weiterhin lässt sich aus den Diagrammen sofort erkennen, dass die Leitfähigkeit darüber hinaus auch abhängig von der Konzentration des Nichtelektrolyten ist. Die Konzentration des Nichtelektrolyten führt quasi zu einer Parallelverschiebung der jeweiligen Graphen zueinander.

Sowohl in Figur 1 als auch in Figur 2 wurde jeweils mittels einer fetten Linie ein Zielwert für die Leitfähigkeit angedeutet. Der Zielwert ist so eingestellt, dass bei korrektem Vorliegen des Nichtelektrolyten, nämlich einer Konzentration des Nichtelektrolyten von 100%, auch 100 % des Elektrolyten erreicht werden. Entsprechend liegt der Zielwert in Figur 1 ungefähr bei 13,1 mS/cm und in Figur 2 bei ca. 12 mS/cm.

Aus den Diagrammen der Figuren 1 und 2 ergibt sich jedoch sofort aus der Kurvenschar, dass bei geringeren Konzentrationen des Nichtelektrolyten beim Erreichen des Zielwertes die gewünschte Elektrolytkonzentration von 100 % noch bei weitem nicht erreicht ist. Aus Figur 1 kann beispielsweise entnommen werden, dass beispielsweise bei einer Konzentration von 0 % Glukose die Leitfähigkeit der medizinischen Flüssigkeit derart beeinflusst wird, dass bei Erreichen des Zielwertes der Leitfähigkeit lediglich 90 % des gelösten Elektrolyten vorliegen (entsprechend nur ca. 126 mmol/l). Auch aus Figur 2 ergibt sich ein ähnliches Bild. Hier wird beispielsweise bei Vorliegen von 0 % Icodextrin beim Erreichen des Zielwertes für die Leitfähigkeit nur eine Elektrolytkonzentration von ca. 83 % erreicht.

Entsprechend führt die Messung der Leitfähigkeit einer Dialysat allein nur dann zu einem korrekten Ergebnis bezüglich der Elektrolytkonzentration, wenn gleichzeitig sichergestellt wird, dass die Nichtelektrolytkonzentration dem vorgegebenen Wert entspricht.

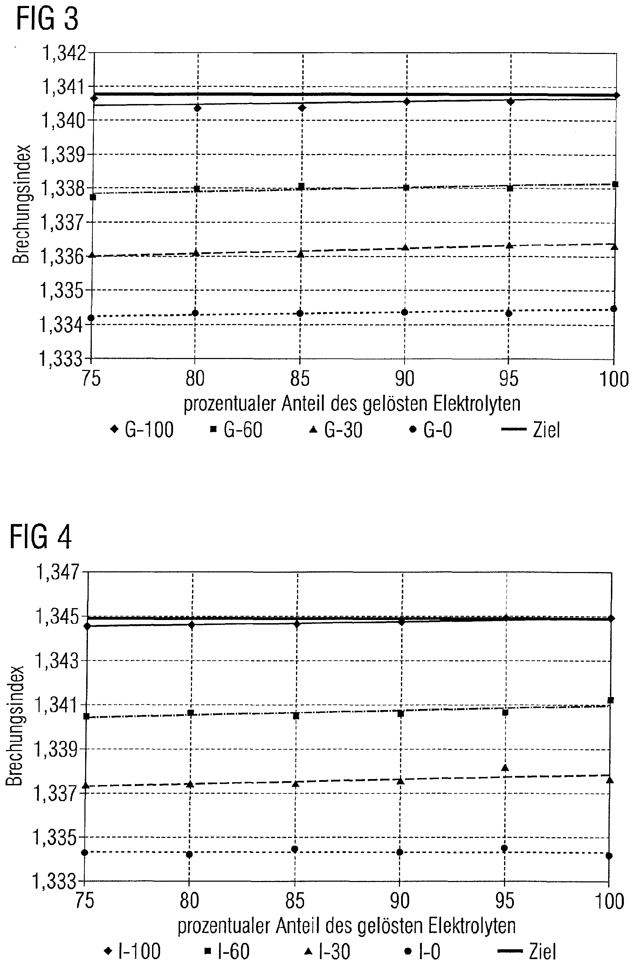

In den Figuren 3 und 4 wurden Messungen des Brechungsindex der medizinischen Flüssigkeit in Abhängigkeit vom prozentualen Anteil des gelösten Elektrolyten bei verschiedenen prozentualen Anteilen des gelösten Nichtelektrolyten durchgeführt. Konkret wurden in den Figuren 3 und 4 die gleichen Konzentrationen für den Elektrolyten (NaCl) und die Nichtelektrolyten (Glukose in Figur 3 beziehungsweise Icodextrin in Figur 4) verwendet, wie in den Figuren 1 und 2. Der Brechungsindex wurde jeweils mit einem handelsüblichen Laborrefraktometer der Bezeichnung "DR6000" des Herstellers Krüss gemessen.

Wie sich auf den ersten Blick aus den Figuren 3 und 4 sofort ergibt, ist der prozentuale Anteil des gelösten Elektrolyten in der jeweiligen getesteten medizinischen Flüssigkeit nicht ausschlaggebend für den gemessenen Brechungsindex. Der Brechungsindex der jeweiligen medizinischen Flüssigkeit ist vielmehr im Wesentlichen unabhängig von der Elektrolytkonzentration - zumindest im betrachteten Konzentrationsbereich, welcher dem physiologisch richtigen Bereich entspricht.

Die leicht sichtbare Steigerung der Kurven ist im Wesentlichen zu vernachlässigen. Aus den Figuren 3 und 4 ist aber auch zu entnehmen, dass der Brechungsindex mit der Konzentration des Nichtelektrolyten zunimmt. Ein Zielwert, welcher in den Figuren 3 und 4 wiederum durch die fett durchgezogene Linie angegeben wird, ist hier durch die korrekte beziehungsweise vorgegebene Konzentration des Nichtelektrolyten festgelegt.

Entsprechend kann aus einer Messung des Brechungsindex unabhängig von der gelösten Elektrolytkonzentration auf die konkrete Konzentration des Nichtelektrolyten in der medizinischen Flüssigkeit geschlossen werden. Eine Aussage über die Elektrolytkonzentration ist jedoch mittels der Messung des Brechungsindex nicht möglich.

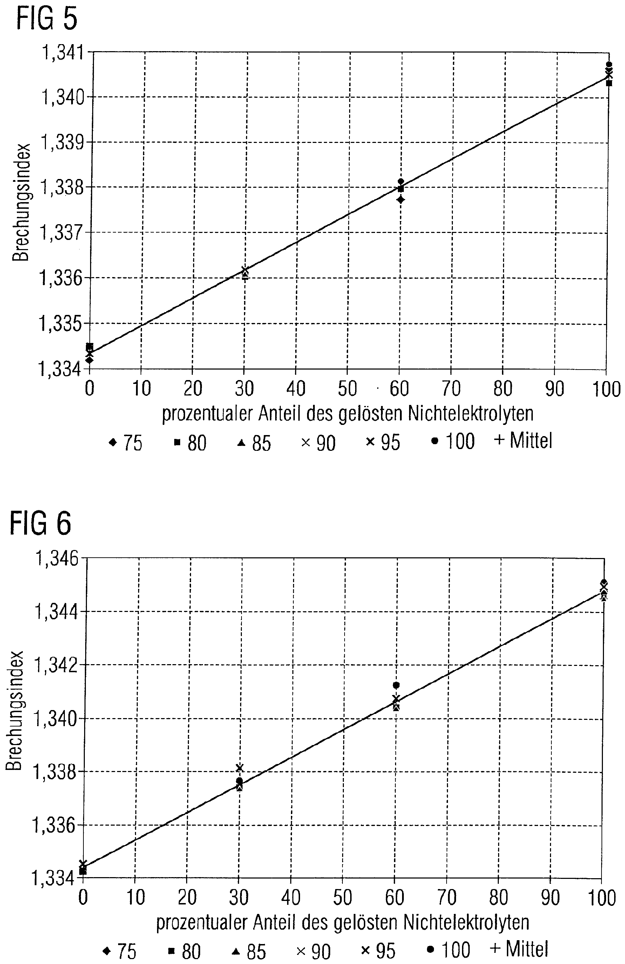

In den Figuren 5 und 6 sind weitere Messungen des Brechungsindex, nun in Abhängigkeit vom prozentualen Anteil des gelösten Nichtelektrolyten in der medizinischen Flüssigkeit, bei verschiedenen prozentualen Anteilen des Elektrolyten gezeigt. Auch für diese Messungen wurden die gleichen Lösungen wie in den oben genannten Messungen verwendet, wobei in Figur 5 wiederum Glukose als Nichtelektrolyt verwendet wird, und in Figur 6 Icodextrin. Der Elektrolyt war in beiden Fällen NaCl.

Es ergibt sich aus den Figuren 5 und 6 sofort, dass ein proportionaler Zusammenhang zwischen dem prozentualen Anteil des gelösten Nichtelektrolyten und dem Brechungsindex besteht, wohingegen die Konzentration des Elektrolyten - zumindest im physiologisch relevanten Konzentrationsbereich - keinen Einfluss auf die Messung des Brechungsindex hat.

Gemäß dem vorgeschlagenen Verfahren wird entsprechend eine Bestimmung mindestens einer ersten physikalischen Größe der medizinischen Flüssigkeit, welche zur eindeutigen Bestimmung des Anteils an Nichtelektrolyten verwendet werden kann, durchgeführt. Bei dieser Bestimmung der ersten physikalischen Größe handelt es sich bevorzugt um den Brechungsindex, welcher, wie sich beispielsweise aus den Figuren 3 bis 6 ergibt, tatsächlich einen eindeutigen Rückschluss auf die Konzentrationen des Nichtelektrolyten in der medizinischen Flüssigkeit ermöglicht.

Über die Bestimmung des Brechungsindex kann direkt auf den Anteil des Nichtelektrolyten geschlossen werden.

Simultan zur Bestimmung des Brechungsindex wird eine zweite Messung durchgeführt, welche zur Bestimmung einer zweiten physikalischen Größe dient, die eine Bestimmung des Anteils an Elektrolyten ermöglicht. Bevorzugt wird hier die Leitfähigkeit gemessen. Wie sich beispielsweise aus den Figuren 1 und 2 ergibt, kann bei Kenntnis der Konzentration des Nichtelektrolyten mittels der Leitfähigkeitsmessung auf die Konzentration des Elektrolyten geschlossen werden.

Der Einfluss des Nichtelektrolyten auf die Bestimmung der Konzentration des Elektrolyten kann auf diese Weise herausgerechnet werden. In einer Alternative können die jeweiligen Werte für die Konzentration des Elektrolyten aus einer gespeicherten Tabelle/Datenbank ausgelesen werden, wobei hier für unterschiedliche Konzentrationen des Nichtelektrolyten Leitfähigkeitswerte für bestimmte Konzentrationen von Elektrolyten hinterlegt sind.

Auf Grundlage der beiden gemessenen physikalischen Größen, nämlich des Brechungsindex und der Leitfähigkeit, kann entsprechend festgestellt werden, ob der Anteil an Elektrolyt und an Nichtelektrolyt in der betrachteten medizinischen Flüssigkeit dem jeweiligen Vorgabewert entspricht. Diese Bestimmung wird bevorzugt automatisch durchgeführt.

Entsprechend kann im Gegensatz zur alleinigen Messung der Leitfähigkeit bei einer kombinierten Messung der beiden Größen eine Überwachung der Zusammensetzung der medizinischen Flüssigkeit erreicht werden.

Bei Verwendung in einem medizinischen Gerät, beispielsweise einer Dialysevorrichtung, kann durch die Bestimmung des Brechungsindex der medizinischen Flüssigkeit überprüft werden, ob sich die Nichtelektrolytkonzentration in einem bestimmten Zielwertbereich befindet. Liegt der Brechungsindex in diesem Zielwertbereich, so weist dies darauf hin, dass die verwendete medizinische Flüssigkeit die gewünschte Nichtelektrolytkonzentration aufweist. Die dann noch offene Frage nach der Elektrolytkonzentration wird dann durch die Leitfähigkeitsmessung eindeutig bestimmt.

Entsprechend kann in dieser Konstellation, insbesondere wenn ein erstes Konzentrat beispielsweise in Trockenform oder in Flüssigform, welches den Nichtelektrolyten umfasst, und ein zweites Konzentrat, ebenfalls in Flüssigform oder in Trockenform, welches den Elektrolyten umfasst, in die medizinische Flüssigkeit gemischt wird, sichergestellt werden, dass sich sowohl die Konzentration des Nichtelektrolyten über die Messung des Brechungsindex, als auch die Konzentration des Elektrolyten über Messung der Leitfähigkeit im Zielwertbereich befinden.

Durch die eindeutige Feststellung der richtigen Lösungszusammensetzung kann entsprechend eine erhöhte Patientensicherheit gewährleistet werden.

Eine Möglichkeit zur einfachen Überwachung einer medizinischen Vorrichtung zur Vorbereitung von medizinischen Flüssigkeiten wird dann über die Hinterlegung von Zielwertbereichen für den Brechungsindex sowie für die Leitfähigkeit der medizinischen Flüssigkeit gegeben. Wandern die gemessenen Werte des Brechungsindex sowie der Leitfähigkeit aus den vorgegebenen Zielwertbereichen, wird die Behandlung beendet oder unterbrochen.

In einer bevorzugten Variante wird weiterhin auch die Temperatur der jeweiligen gemessenen medizinischen Flüssigkeit gemessen, um die sich aus Temperaturänderungen ergebenden Variationen der Messwerte des Brechungsindex sowie der Leitfähigkeit bei der Bestimmung, ob sich die medizinische Flüssigkeit im vorgegebenen Bereich bewegt, zu berücksichtigen.

Es ist jedoch auch möglich, auf Grundlage der durchgeführten Messungen auf einen potentiellen Fehlerfaktor zu schließen, beispielsweise auf eine zu geringe Hinzudosierung des Nichtelektrolyten oder des Elektrolyten.

Über eine entsprechende Auswertung lässt sich nämlich bei Vorliegen der entsprechenden Datenlage sowohl die Konzentration des Elektrolyten als auch die Konzentration des Nichtelektrolyten bestimmen.

Wie sich aus den Figuren 5 und 6 ergibt, ist der Brechungsindex linear abhängig von der Konzentration des Nichtelektrolyten in der medizinischen Flüssigkeit. Hieraus ergibt sich:

$$(1) \qquad RI = RI_0 + x_{NE}\, m_{RI}$$

Hierbei ist RI der Brechungsindex der medizinischen Flüssigkeit, $RI_0$ eine Nullpunktverschiebung, welche durch die verwendete Flüssigkeit bestimmt wird, $x_{NE}$ die Konzentration beziehungsweise der Auflösungsgrad des Nichtelektrolyten in der medizinischen Flüssigkeit und $m_{RI}$ die Steigung der jeweiligen Messkurve in den Figuren.

Hieraus lässt sich der Auflösungsgrad beziehungsweise die Konzentration des Nichtelektrolyten bestimmen zu:

$$(2) \qquad x_{NE} = (RI - RI_0)\, /\, m_{RI}$$

Auch die Leitfähigkeit lässt sich entsprechend linearisieren, wie sich beispielsweise aus den Figuren 1 und 2 ergibt, da eine lineare Abhängigkeit zwischen der Elektrolytkonzentration und der Nichtelektrolytkonzentration auf der einen Seite und der Leitfähigkeit auf der anderen Seite besteht. Hierbei ist für eine exakte Bestimmung jedoch zu beachten, dass die jeweiligen Geraden in den Figuren 1 und 2 nicht genau parallel zueinander verschoben sind, sondern eine leicht unterschiedliche Steigung aufweisen. Daher gilt für die Leitfähigkeit:

$$(3) \qquad LF = LF_0(x_{NE}) + x_E\, x\, m_{LF}\,(x_{NE})$$

Dabei ist LF die Leitfähigkeit der medizinischen Flüssigkeit, $LF_0(x_{NE})$ die durch den Nichtelektrolyten verursachte Nullpunktverschiebung, $x_E$ die Konzentration beziehungsweise der Auflösungsgrad des Elektrolyten und $m_{LF}(x_{NE})$ die von der Konzentration des Nichtelektrolyten abhängige Steigung der gemessenen Kurve.

Damit ergibt sich für die Konzentration des Elektrolyten beziehungsweise dessen Auflösungsgrad:

$$(4) \qquad x_E = (LF - LF_0)\, (x_{LME})\, /\, m_{LF}(x_{ME})$$

Da jedoch der Effekt der Nichtelektrolytkonzentration auf die Steigung der Leitfähigkeit nicht sehr groß - beziehungsweise vernachlässigbar - ist, wird zur Vereinfachung mit einer mittleren Steigung gerechnet:

$$(5) \qquad x_E = (LF - LF_0)\,(x_{ME})\,/\,m_{LF}$$

Hieraus ergibt sich die entsprechende Abschätzung sowohl für den Auflösungsgrad beziehungsweise die Konzentration des Nichtelektrolyten (vergleiche Gleichung (2)), sowie für die Konzentration beziehungsweise den Auflösungsgrad des Elektrolyten (vergleiche Gleichung (5)).

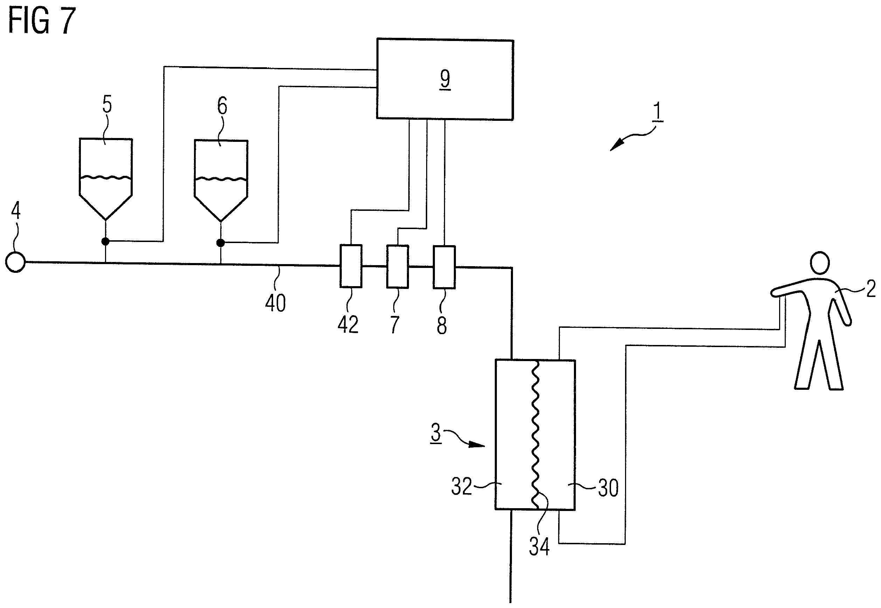

Figur 7 zeigt schematisch eine Hämodialysevorrichtung 1 gezeigt, mittels welcher ein schematisch angedeuteter Patient 2 dialysiert wird. Ein Dialysator 3 ist vorgesehen, welcher eine Patientenseite 30 und eine Dialysatseite 32 aufweist, wobei die beiden Kammern über die Dialysemembran 34 voneinander getrennt sind.

Das Dialysat wird online zubereitet und es wird Wasser von einem Wasseranschluss 4 mit einem Elektrolytkonzentrat aus einer entsprechenden Elektrolytdosiervorrichtung 5 und einem Nichtelektrolytkonzentrat aus einer entsprechenden Nichtelektrolytdosiervorrichtung 6 gemischt. Die Elektrolytdosiervorrichtung 5 sowie die Nichtelektrolytdosiervorrichtung 6 werden jeweils automatisch angesteuert - beispielsweise über eine Auswertungsvorrichtung 9 - und dosieren entsprechend eine solche Menge an Elektrolytkonzentrat sowie an Nichtelektrolytkonzentrat in das Wasser, dass die geforderten Konzentrationen an Elektrolyt sowie an Nichtelektrolyt in dem entstehenden Dialysat erreicht werden.

Ein Brechungsindexsensor 7 ist nach den beiden Dosiervorrichtungen 5, 6 in der Leitung 40, mittels welcher das Dialysat transportiert wird, vorgesehen. Der Brechungsindexsensor 7 ist dazu vorgesehen, den Brechungsindex des Dialysats so zu bestimmen, dass hierüber direkt auf die Konzentration des Nichtelektrolyten im Dialysat geschlossen werden kann. Diese Auswertung wird über die Steuervorrichtung 9 erreicht, an welche die entsprechenden Signale des Brechungsindexsensor 7 geleitet wird. Die entsprechenden mathematischen Zusammenhänge wurden bereits oben ausgeführt. Insbesondere besteht ein linearer Zusammenhang zwischen dem Brechungsindex und der Konzentration des Nichtelektrolyten in dem Dialysat.

Weiterhin ist ein Leitfähigkeitssensor 8 vorgesehen, mittels welchem die Leitfähigkeit des hergestellten Dialysats gemessen werden kann. Wie bereits ebenfalls oben beschrieben, kann aus der Leitfähigkeit des Dialysats auf die Konzentration des Elektrolyten geschlossen werden, wenn die Konzentration des Nichtelektrolyten berücksichtigt wird. Da die Konzentration des Nichtelektrolyten jedoch bereits auf Grundlage der Messung des Brechungsindexsensors 7 bekannt ist, kann in der Auswertungsvorrichtung 9 entsprechend auch auf die Konzentration des Elektrolyten im Dialysat geschlossen werden.

In einer bevorzugten Variante ist weiterhin noch ein Temperatursensor 42 in der Leitung 40 vorgesehen, mittels welchem die Temperatur des Dialysats festgestellt werden kann, da sich der Brechungsindex und/oder die Leitfähigkeit des Dialysats mit der Temperatur ändern können.

Die in Figur 7 gezeigte Hämodialysevorrichtung ist lediglich schematisch gezeigt. Es ist für den Fachmann sofort zu erkennen, dass die beschriebene, simultane Messung des Brechungsindex und der Leitfähigkeit nicht nur in Hämodialysevorrichtungen, sondern auch in Hämofiltrationsvorrichtungen zur Überwachung der Substitutionsflüssigkeit, in Hämodiafiltrationsvorrichtungen zur Überwachung der Konzentrationen des Dialysats sowie der Überwachung der Substitutionsflüssigkeit, sowie in Peritonealdialysevorrichtungen zur Überwachung des Dialysats verwendet werden kann.

Auch zur Überwachung beziehungsweise Bestimmung der Anteile beziehungsweise Konzentrationen von Elektrolyten und Nichtelektrolyten in medizinischen Flüssigkeiten in anderen medizinischen Kontexten kann die vorliegende Erfindung verwendet werden.

Die medizinische Flüssigkeit, insbesondere das Dialysat, kann auf unterschiedliche Weisen hergestellt werden. Insbesondere kann sie durch Mischen von Trockenkonzentrat mit einer Grundflüssigkeit, beispielsweise Wasser, hergestellt werden, wobei in dem Trockenkonzentrat entweder die Elektrolyte und die Nichtelektrolyte bereits vorgemischt vorliegen, oder aber zwei unterschiedliche Trockenkonzentrate miteinander in dem gewünschten Verhältnis gemischt werden, wobei in dem einen Trockenkonzentrat die Elektrolyte und in dem anderen Trockenkonzentrat die Nichtelektrolyte vorgesehen sind. Eine Mischung der medizinischen Flüssigkeit kann weiterhin auch über das Hinzufügen von Flüssigkonzentraten, entweder kombinierten Flüssigkonzentraten oder aber getrennten Flüssigkonzentraten, durchgeführt werden. Auch eine Kombination von einem Trockenkonzentrat, beispielsweise für die Elektrolyte, und einem Flüssigkonzentrat, beispielsweise für die Nichtelektrolyte, beziehungsweise andersherum, kann ebenfalls durchgeführt werden. Auch eine Mischung verdünnter Lösungen, in welchen Elektrolyte und Nichtelektrolyte getrennt vorliegen, kann zu einer medizinischen Flüssigkeit führen.

In Figur 7 ist es neben der Überwachung der Konzentrationen der Elektrolyte beziehungsweise der Nichtelektrolyte über die Brechungsindexmessung, Leitfähigkeitsmessung sowie ggf. Temperaturmessung möglich, die Konzentrationen über einen Regelkreislauf zu beeinflussen. Dazu wird von der Auswertungsvorrichtung 9 bei einer Abweichung der gemessenen Konzentrationen des Elektrolyten beziehungsweise des Nichtelektrolyten gegenüber einer gewünschten Konzentration die entsprechende Elektrolytdosiervorrichtung 5 beziehungsweise Nichtelektrolytdosiervorrichtung 6 in ihrem Dosierverhalten beeinflusst. Ist beispielsweise die Konzentration des Nichtelektrolyten zu niedrig, so wird die Nichtelektrolytdosiervorrichtung 6 angewiesen, eine höhere Menge an Nichtelektrolytkonzentrat abzugeben. Gleiches kann auch für die Elektrolytdosiervorrichtung 5 vorgenommen werden.

In einer Variante werden in der Auswertungsvorrichtung 9 Zielwerte für die Elektrolytkonzentration sowie die Nichtelektrolytkonzentration, beziehungsweise Zielwertbereiche für die jeweiligen Konzentrationen gespeichert. Wenn die gemessenen Werte für die Elektrolytkonzentrationen sowie die Nichtelektrolytkonzentrationen außerhalb den Zielwertbereichen liegen, so wird die Dialyse unterbrochen oder abgebrochen.

Die Abhängigkeit des Brechungsindex von der Nichtelektrolytkonzentration ist relativ klein, so dass die vorliegende Erfindung besonders zur Herstellung von Lösungen beziehungsweise medizinischen Flüssigkeiten mit hohen Nichtelektrolytkonzentrationen geeignet ist. Das vorliegende Verfahren beziehungsweise die vorliegende Vorrichtung ist deshalb besonders zur Überwachung der Herstellung von medizinischen Flüssigkeiten beziehungsweise Dialysaten für die Peritonealdialyse geeignet, weil diese Dialysate mit 42,5 g/l Glukose beziehungsweise 75 g/l Icodextrin eine hohe Nichtelektrolytkonzentration aufweisen.

Bei der Verwendung des Verfahrens beziehungsweise der Vorrichtung für die Hämodialyse ist der Effekt der Brechungsindexmessung für Nichtelektrolyte relativ gering, da in solchen Dialysaten üblicherweise Konzentrationen von Glukose im Bereich von 1 g/l verwendet werden. Diese relativ geringen Konzentrationen haben jedoch nur einen geringen Einfluss auf die Leitfähigkeit, so dass die herkömmliche Dialyse auch mit Leitfähigkeitsmessungen alleine auskommen kann. Bei Verwendung von Dialyseverfahren beziehungsweise Dialysevorrichtungen, welche jedoch höhere Nichtelektrolytkonzentrationen verwenden, beispielsweise bei der Zumischung von Glukose für spezielle Glukoseprofile beziehungsweise für eine Bolusgabe von Glukose, kann die vorliegende Vorrichtung beziehungsweise das vorliegende Verfahren besonders vorteilhaft eingesetzt werden.

Bezuaszeichenliste

1 Hämodialysevorrichtung
2 Patient
3 Dialysator
30 Patientenseite
32 Dialysatseite
34 Membran
4 Wasseranschluss
40 Leitung
42 Temperatursensor
5 Elektrolytdosiervorrichtung
6 Nichtelektrolytdosiervorrichtung
7 Brechungsindexsensor
8 Leitfähigkeitssensor
9 Auswertungsvorrichtung

**Patentansprüche**

1. Verfahren zur Bestimmung der Zusammensetzung medizinischer Flüssigkeiten bezüglich ihres Anteils an Elektrolyten und Nichtelektrolyten, umfassend die Schritte:

   - Bestimmen mindestens einer ersten physikalischen Größe der medizinischen Flüssigkeit zur eindeutigen Bestimmung des Anteils an Nichtelektrolyten in der medizinischen Flüssigkeit;
   - simultanes Bestimmen mindestens einer zweiten physikalischen Größe der medizinischen Flüssigkeit zur Bestimmung des Anteils an Elektrolyten in der medizinischen Flüssigkeit; und
   - Bestimmen des Anteils an Elektrolyten und Nichtelektrolyten in der medizinischen Flüssigkeit auf Grundlage der ersten und der zweiten physikalischen Größe,

   wobei aus der ersten physikalischen Größe der Anteil des Nichtelektrolyten eindeutig bestimmt wird und diese Bestimmung des Anteils des Nichtelektrolyten in die Bestimmung des Anteils des Elektrolyten auf Grundlage der zweiten physikalischen Größe mit eingeht, wobei bevorzugt der Effekt des Nichtelektrolyten auf die Messung der zweiten physikalischen Größe bei der Bestimmung des Anteils an Elektrolyten berücksichtigt wird, **dadurch gekennzeichnet, dass** die erste physikalische Größe der Brechungsindex der medizinischen Flüssigkeit ist.

2. Verfahren gemäß Anspruch 1, wobei die zweite physikalische Größe die elektrische Leitfähigkeit der medizinischen Flüssigkeit ist.

3. Verfahren gemäß einem der vorstehenden Ansprüche, wobei ein Zielwert oder ein Zielwertbereich für die erste physikalische Größe und/oder ein Zielwert oder ein Zielwertbereich für die zweite physikalische Größe vorbestimmt wird, und bei einer Abweichung der gemessenen ersten physikalischen Größe und/oder der zweiten physikalischen Größe von dem jeweiligen vorgegebenen Zielwert beziehungsweise Zielwertbereich ein Alarm ausgelöst wird und/oder eine Behandlung abgebrochen wird.

4. Verfahren gemäß einem der vorstehenden Ansprüche, wobei die Temperatur der medizinischen Flüssigkeit gemessen wird und die Temperatur bei der Bestimmung des Anteils an Elektrolyten und Nichtelektrolyten auf Grundlage der ersten und zweiten physikalischen Größe berücksichtigt wird.

5. Verfahren zum Betrieb einer Dialysevorrichtung, wobei der Anteil an Elektrolyten und Nichtelektrolyten in dem Dialysat mittels des Verfahrens gemäß einem der vorstehenden Ansprüche bestimmt wird.

6. Vorrichtung zur Bestimmung der Zusammensetzung medizinischer Flüssigkeiten bezüglich ihres Anteils an Elektrolyten und Nichtelektrolyten, umfassend
eine erste Messvorrichtung (7) zum Messen einer ersten physikalischen Größe der medizinischen Flüssigkeit, welche in einem eindeutigen Verhältnis zu dem Anteil des Nichtelektrolyten in der medizinischen Flüssigkeit steht;
eine zweite Messvorrichtung (8) zur simultanen Bestimmung mindestens einer zweiten physikalischen Größe der medizinischen Flüssigkeit, welche in einem Verhältnis zu dem Anteil an Elektrolyten in der medizinischen Flüssigkeit steht; und
eine Auswertungsvorrichtung (9) zum Bestimmen des Anteils an Elektrolyten und Nichtelektrolyten in der medizinischen Flüssigkeit auf Grundlage der Messwerte der ersten Messvorrichtung (7) und der zweiten Messvorrichtung (8),
wobei die Auswertungsvorrichtung (9) ferner derart konfiguriert ist, dass aus der ersten physikalischen Größe der Anteil des Nichtelektrolyten eindeutig bestimmt wird und diese Bestimmung des Anteils des Nichtelektrolyten in die Bestimmung des Anteils des Elektrolyten auf Grundlage der zweiten physikalischen Größe mit eingeht, wobei bevorzugt der Effekt des Nichtelektrolyten auf die Messung der zweiten physikalischen Größe bei der Bestimmung des Anteils an Elektrolyten berücksichtigt wird,
**dadurch gekennzeichnet, dass**
die erste Messvorrichtung eingerichtet ist als erste physikalische Messgröße den Brechungsindex der medizinischen Flüssigkeit zu messen.

7. Vorrichtung gemäß Anspruch 6, wobei die zweite Messvorrichtung eingerichtet ist als zweite physikalische Messgröße die elektrische Leitfähigkeit der medizinischen Flüssigkeit zu messen.

8. Vorrichtung gemäß Anspruch 6 oder 7, wobei ein Temperatursensor (42) zur Messung der Temperatur der medizinischen Flüssigkeit vorgesehen ist.

9. Dialysevorrichtung, bevorzugt Hämodialysvorrichtung, Hämofiltrationsvorrichtung, Hämodiafiltrationsvorrichtung oder Peritonealdialysevorrichtung, umfassend eine Vorrichtung gemäß einem der Ansprüche 6 bis 8 zum Bestimmen des Anteils an Elektrolyten und Nichtelektrolyten in dem Dialysat und/oder der Substitutionsflüssigkeit.

## Claims

1. A method for determining the composition of medical liquids with regard to their fraction of electrolytes and non-electrolytes, comprising the steps of:

- determining at least one first physical parameter of the medical liquid for unambiguously determining the fraction of non-electrolytes in the medical liquid;
- simultaneously determining at least a second physical parameter of the medical liquid for determining the fraction of electrolytes in the medical liquid; and
- determining the fraction of electrolytes and non-electrolytes in the medical liquid based on the first and second physical parameter,

wherein the fraction of the non-electrolyte is unambiguously determined from the first physical parameter, and this determination of the fraction of the non-electrolyte is included in determining the fraction of the electrolyte on the basis of the second physical parameter, wherein, preferably, the effect of the non-electrolyte on the measurement of the second physical parameter is taken into account in determining the fraction of electrolytes
**characterized in that**
the first physical parameter is the refractive index of the medical liquid.

2. The method according to claim 1, wherein the second physical parameter is the electrical conductivity of the medical liquid.

3. The method according to any one of the preceding claims, wherein a target value or target value range for the first physical parameter and/or a target value or target value range for the second physical parameter is predetermined, and an alarm is triggered and/or treatment is discontinued, when the measured first physical parameter and/or the second physical parameter deviates from the respective predetermined target value or target value range.

4. A method according to any one of the preceding claims, wherein the temperature of the medical liquid is measured and the temperature is taken into account in determining the fraction of electrolytes and non-electrolyte based on the first and second physical parameter.

5. A method for operating a dialysis apparatus, wherein the fraction of electrolytes and non-electrolytes in the dialysate is determined by means of the method according to anyone of the preceding claims.

6. An apparatus for determining the composition of medical liquids with regard to their fraction of electrolytes and non-electrolytes, which comprises a first measuring apparatus (7) for measuring a first physical parameter of the medical liquid, which is unambiguously related to the fraction of the non-electrolyte in the medical liquid;

a second measuring apparatus (8) for simultaneously determining at least a second physical parameter of the medical liquid, which is related to the fraction of electrolytes in the medical liquid; and

an evaluating apparatus (9) for determining the fraction of electrolytes and non-electrolytes in the medical liquid on the basis of the measured values of the first measuring apparatus (7) and the second measuring apparatus (8),

wherein the evaluating apparatus (9) is further configured such that the fraction of the non-electrolytes is unambiguously determined from the first physical parameter, and this determination of the fraction of the non-electrolytes is included in determining the fraction of the electrolytes on the basis of the second physical parameter, wherein, preferably, the effect of the non-electrolytes on the measurement of the second physical parameter is taken into account in determining the fraction of electrolytes,

**characterized in that**

the first measuring apparatus is configured to measure the refractive index of the medical liquid as first physical parameter.

7. The apparatus of claim 6, wherein the second measuring apparatus is configured to measure the electrical conductivity of the medical liquid as second physical parameter.

8. The apparatus according to any one of the claims 6 to 7, wherein a temperature sensor (42) is provided for measuring the temperature of the medical liquid.

9. Dialysis apparatus, preferably hemodialysis apparatus, hemofiltration apparatus, hemodiafiltration apparatus or peritoneal dialysis apparatus, comprising an apparatus according to any one of the claims 6 to 8 for determining the fraction of electrolytes and non-electrolytes in the dialysate and/or the substitution solution.

**Revendications**

1. Procédé de détermination de la composition de liquides médicaux quant à leur teneur en électrolytes et en non-électrolytes, comprenant les étapes :

   - détermination d'au moins une première grandeur physique du liquide médical en vue de la détermination sans équivoque de la teneur en non-électrolytes dans le liquide médical ;
   - détermination simultanée d'au moins une deuxième grandeur physique du liquide médical en vue de la détermination de la teneur en électrolytes dans le liquide médical ; et
   - détermination de la teneur en électrolytes et en non-électrolytes dans le liquide médical sur la base de la première et de la deuxième grandeur physique,

   dans lequel on détermine sans équivoque la teneur en non-électrolytes à partir de la première grandeur physique et on inclut cette détermination de la teneur en non-électrolytes dans la détermination de la teneur en électrolytes sur la base de la deuxième grandeur physique, l'effet du non-électrolyte sur la mesure de la deuxième grandeur physique étant alors de préférence pris en compte lors de la détermination de la teneur en électrolytes,

   **caractérisé en ce que** la première grandeur physique est l'indice de réfraction du liquide médical.

2. Procédé selon la revendication 1, dans lequel la deuxième grandeur physique est la conductivité électrique du liquide médical.

3. Procédé selon l'une quelconque des revendications précédentes, dans lequel on détermine au préalable une valeur cible ou une plage de valeur cible pour la première grandeur physique et/ou une valeur cible ou une plage de valeur cible pour la deuxième grandeur physique et dans lequel, en présence d'un écart entre la première grandeur physique mesurée et/ou la deuxième grandeur physique et la valeur cible ou plage de valeur cible prédéterminée respective, on déclenche une alarme et/ou on interrompt un traitement.

4. Procédé selon l'une quelconque des revendications précédentes, dans lequel on mesure la température du liquide médical et on prend en compte la température lors de la détermination de la teneur en électrolytes et en non-électrolytes sur la base de la première et de la deuxième grandeur physique.

5. Procédé de fonctionnement d'un dispositif de dialyse, dans lequel on détermine la teneur en électrolytes et en non-électrolytes dans le dialysat au moyen du procédé selon l'une quelconque des revendications précédentes.

6. Dispositif de détermination de la composition de liquides médicaux quant à leur teneur en électrolytes et en non-électrolytes, comprenant

   un premier dispositif de mesure (7) destiné à mesurer une première grandeur physique du liquide médical qui se trouve dans un rapport univoque avec la teneur en non-électrolytes dans le liquide médical ;

   un deuxième dispositif de mesure (8) destiné à la déterminer simultanément au moins une deuxième grandeur physique du liquide médical qui se trouve dans un certain rapport avec la teneur en électrolytes dans le liquide médical ; et

un dispositif d'évaluation (9) destiné à déterminer la teneur en électrolytes et en non-électrolytes dans le liquide médical sur la base des valeurs mesurées du premier dispositif de mesure (7) et du deuxième dispositif de mesure (8),

dans lequel le dispositif d'évaluation (9) est configuré en outre de telle sorte que la teneur en non-électrolytes est déterminée sans équivoque à partir de la première grandeur physique et cette détermination de la teneur en non-électrolytes est incluse dans la détermination de la teneur en électrolytes sur la base de la deuxième grandeur physique, l'effet du non-électrolyte sur la mesure de la deuxième grandeur physique étant alors de préférence pris en compte lors de la détermination de la teneur en électrolytes, **caractérisé en ce que** le premier dispositif de mesure est équipé pour mesurer comme première grandeur physique l'indice de réfraction du liquide médical.

7. Dispositif selon la revendication 6, dans lequel le deuxième dispositif de mesure est équipé pour mesurer comme deuxième grandeur physique la conductivité électrique du liquide médical.

8. Dispositif selon la revendication 6 ou 7, dans lequel un capteur de température (42) est prévu pour mesurer la température du liquide médical.

9. Dispositif de dialyse, de préférence dispositif d'hémodialyse, dispositif d'hémofiltration, dispositif d'hémodiafiltration ou dispositif de dialyse péritonéale, comprenant un dispositif selon l'une quelconque des revendications 6 à 8 afin de déterminer la teneur en électrolytes et en non-électrolytes dans le dialysat et/ou dans le liquide de substitution.

# FIG 1

# FIG 2

## FIG 3

## FIG 4

# FIG 5

prozentualer Anteil des gelösten Nichtelektrolyten

◆ 75    ■ 80    ▲ 85    × 90    × 95    ● 100    + Mittel

# FIG 6

prozentualer Anteil des gelösten Nichtelektrolyten

◆ 75    ■ 80    ▲ 85    × 90    × 95    ● 100    + Mittel

FIG 7

EP 2 833 938 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5762769 A
- EP 1547629 A1
- JP 2012026912 A
- JP H1085573 A